# FASCICULE DE BREVET EUROPEEN

(11) **EP 1 165 214 B1**
(45) Date de publication et mention de la délivrance du brevet: **25.11.2009**
(21) Numéro de dépôt: 01903969.2
(22) Date de dépôt: 26.01.2001
(51) Int. Cl.: B01D 67/00, B01D 69/12, B01D 69/02, B01D 71/42, A61L 33/00

(54) **PROCEDE DE FABRICATION D'UNE MEMBRANE SEMI-PERMEABLE NON THROMBOGENE**
VERFAHREN ZUR HERSTELLUNG EINER TEILDURCHLÄSSIGEN NICHT THROMBOGENE MEMBRAN
METHOD FOR PRODUCING A NON-THROMBOGENIC SEMIPERMEABLE MEMBRANE

(30) Priorité: 27.01.2000 FR 0001065
(43) Date de publication de la demande: 02.01.2002
(73) Titulaire: Gambro Industries SAS, 69330 Meyzieu (FR)
(72) Inventeur: CROST, Thierry, F-01800 St Maurice de Gourdans (FR); RENAUX, Jean-Louis, F-69300 Caluire (FR); THOMAS, Michel, F-69360 Sérezin du Rhône (FR)
(74) Mandataire: Ponzellini, Gianmarco
(86) Numéro de dépôt international: PCT/FR2001/000248
(87) Numéro de publication internationale: WO 2001/054802

(56) Documents cités:
- EP-A- 0 925 826
- US-A- 4 749 619
- US-A- 5 840 190

## Description

La présente invention se situe dans le domaine du traitement du sang et du plasma par circulation extracorporelle, notamment par dialyse, hémofiltration et plasmaphérèse. Elle a pour objet une membrane semi-perméable composite comprenant une membrane semi-perméable support revêtue d'un agent anticoagulant, réduisant le caractère thrombogène du support.

La présente invention a également pour objet des échangeurs pour le traitement du sang ou du plasma par circulation extracorporelle, comprenant cette membrane semi-perméable composite ainsi que des procédés de fabrication de ces échangeurs.

Par membrane semi-perméable, on entend, dans l'ensemble de ce texte, une membrane semi-perméable plane ou un faisceau de fibres creuses semi-perméables. Egalement, par échangeur, on entend, dans l'ensemble de ce texte, un échangeur pour le traitement du sang ou du plasma par circulation extracorporelle qui comprend, d'une manière générale, deux compartiments séparés par une membrane semi-perméable, munis chacun de deux accès, un premier compartiment étant destiné à la circulation du sang ou du plasma du patient, et un second compartiment étant destiné à la circulation de liquide usé.

Le traitement du sang ou du plasma d'un patient urémique nécessite l'utilisation d'un circuit extracorporel de sang connecté à un échangeur. Le sang, dans le circuit extracorporel, est soumis à un risque thrombotique majeur qu'il faut prévenir par une anticoagulation efficace. C'est pourquoi, bien que les matériaux utilisés pour la fabrication des échangeurs soient choisis pour être aussi biocompatibles que possible de façon que, en particulier, les réactions de coagulation ne se produisent pas ou à des niveaux relativement bénins, on injecte habituellement, dans le sang du patient, un agent anti-coagulant, généralement de l'héparine (fractionnée ou non).

La quantité d'héparine injectée au patient varie classiquement de 7.500 à 11.000 unités internationales (UI) par séance de traitement, voire plus dans certains cas.

Dans la pratique courante, l'anticoagulation du sang pose rarement de difficultés. Chez certains patients cependant l'héparine peut provoquer des réactions secondaires indésirables, en particulier des hémorragies. D'autres effets secondaires rapportées dans la littérature sont des thrombocytopénies (chute du nombre de plaquettes), des allergies, des ostéoporoses (constatées dans le cas d'une administration prolongée d'héparine), des augmentations des transaminases (enzymes hépatiques) et des hyperlipidémies. La sensibilité à l'héparine est variable d'un patient à un autre et, dans le temps, pour un patient donné.

Pour les patients à haut risque hémorragique, il existe une méthode de traitement extracorporel du sang sans anticoagulant qui consiste à prévenir la thrombose du sang dans le circuit de circulation extracorporelle et dans le dialyseur grâce à des rinçages fréquents du circuit et du dialyseur avec du sérum physiologique pour en chasser les thrombis en formation. Cette méthode implique des débits élevés de circulation du sang qui ne sont pas toujours tolérés par les patients.

En outre, ce traitement sans anticoagulant, qui est techniquement difficile, nécessite une formation spécifique des soignants, le respect rigoureux du protocole opératoire, une surveillance stricte de la dialyse pour prévenir les incidents, le dépistage précoce des incidents pour pouvoir les surmonter.

Dans le but d'éviter l'injection d'anticoagulant dans le circuit extracorporel de sang, de nombreux travaux de recherche ont été entrepris afin d'améliorer l'hémocompatibilité des membranes semi-perméables en fixant de l'héparine sur la surface de la membrane destinée à être mise en contact avec le sang, à l'aide d'une liaison ionique ou covalente (voir J. Pelissié « Héparinisation de surface » dans RBM (1994) 16, 7, 290-291).

Les techniques décrites de fixation ionique de l'héparine visent à créer des groupements amines quaternaires sur la surface de la membrane semi-perméable à traiter. L'héparine, qui est chargée négativement par ses groupements sulfates et carboxyliques, peut réagir avec des groupements amines quaternaires. Mais, il a été constaté un relargage rapide d'héparine dû à la faiblesse de la liaison intermoléculaire, ce qui présente l'inconvénient de réduire le caractère antithrombogénique des surfaces destinées à être en contact avec le sang et de favoriser l'apparition d'effets secondaires indésirables dus à l'héparine libérée. Pour compenser le relargage continu d'héparine, des quantités importantes de cet anticoagulant doivent être fixées sur la membrane, ce qui a pour conséquence de rendre difficile la maîtrise de l'anticoagulation du sang du patient et d'avoir un coût élevé.

Les techniques décrites de fixation covalente font intervenir des réactions chimiques spécifiques et complexes pour permettre l'accrochage de l'héparine sur les surfaces des matériaux destinées à être mises en contact avec le sang. Cependant ces techniques, parce qu'elles causent une modification substantielle de la nature chimique des matériaux, ne sont généralement pas appropriées aux membranes semi-perméables car elles risqueraient de provoquer une diminution importante des propriétés fondamentales de ces membranes, telles que les capacités de transferts diffusifs et convectifs et la capacité d'adsorption des substances indésirables. En outre, il existe un risque de diminution de l'antithrombogénicité par inactivation de l'héparine.

Le seul échangeur commercialisé à cette date comprenant une membrane semi-perméable traitée avec un agent anticoagulant afin de réduire son caractère thrombogène est l'hémofiltre de dénomination commerciale DURAFLO de la société BAXTER. Cette membrane semi-perméable est à base de polysulfone.

Certaines membranes semi-perméables constituées essentiellement d'un copolymère d'acrylonitrile et d'au moins un monomère anionique ou anionisable, oléfiniquement insaturé, comme le copolymère fabriqué par HOSPAL sous la dénomination commerciale AN69 sont reconnues pour être nettement moins thrombogéniques que les membranes cellulosiques. La mise en oeuvre de dialyse sans héparine avec ces membranes nécessite cependant des débits élevés de circulation du sang [au moins 400 ml/min selon l'article « Heparin-free hemodialysis with a polyacrylonitrile membrane », de Robert H. Barth et al., Vol. XXXV Trans Am Soc Artif Intern Organs (1989)], ce qui n'est pas toujours toléré par tous les patients.

Un premier objectif de la présente invention est de réduire le caractère thrombogène des membranes semi-perméables constituées essentiellement par un copolymère d'acrylonitrile et d'au moins un monomère anionique et anionisable en fixant, sur ces membranes, un agent anticoagulant, tout en maintenant à un niveau acceptable les autres propriétés fondamentales de ces membranes, à savoir leur capacité de transferts diffusifs et convectifs (perméabilité à l'eau, perméabilité sélective aux molécules de masse moléculaire en poids inférieure à celle de l'albumine), leur capacité d'adsorption des substances indésirables et leurs propriétés mécaniques.

Un second objectif de la présente invention est de fixer, de façon stable, à la surface de membranes semi-perméables constituées essentiellement par un copolymère d'acrylonitrile et d'au moins un monomère anionique et anionisable, un agent anticoagulant pouvant exercer son activité anticoagulante sans être élué et relargué rapidement dans le sang ou le plasma au cours du traitement par circulation extracorporelle.

Un troisième objectif de la présente invention est de réduire la quantité d'agent anticoagulant injectée au patient au cours d'une séance de traitement extracorporel de sang mise en oeuvre au moyen d'un échangeur muni d'une membrane semi-perméable constituée essentiellement par un copolymère d'acrylonitrile et d'au moins un monomère anionique et anionisable sur laquelle est fixé un agent anticoagulant.

Pour atteindre ces objectifs, on propose conformément à la présente invention, un procédé selon la revendication 1.

Conformément à l'invention, les chaînes du polymère cationique présentent un encombrement stérique suffisamment important pour ne pas traverser la membrane semi-perméable support, ce qui permet d'obtenir que ces chaînes se fixent essentiellement à la surface de la membrane, par liaison ionique. Dès lors, la quantité de polymère cationique nécessaire pour traiter la membrane semi-perméable support est modérée puisque l'on ne recherche pas un traitement massique de la membrane somi-perméable support avec pénétration du polymère cationique dans les pores.

De préférence, la polymère cationique est préparé par ultrafiltration au moyen d'une membrane semi-perméable qui est identique à la membrane semi-perméable support ou qui possède un seuil de coupure égal ou supérieur à celui de la membrane semi-perméable support, afin d'éliminer les chaînes de polymère cationique pouvant traverser la membrane semi-perméable support.

La membrane semi-perméable composite obtenue selon l'invention présente trois avantages majeurs :
- premièrement, la fixation par liaison ionique de l'agent anti-coagulant se fait quasiment uniquement à la surface de la membrane smi-perméable. Dès lors, l'agent anticoagulant fixé, qui est accessible aux protéines de la coagulation, peut exercer une activité anti-coagulante efficace au cours d'une séance de traitement de sang et de plasma par circulation extracorporelle.
- deuxièmement, le temps de coagulation (TCK pour Temps de Céphaline Activée) d'un sang ou plasma purifié à l'aide de la membrane semi-perméable composite est assez voisin, voire équivalent à celui d'un sang ou d'un plasma normal, non additionné d'agent anticoagulant.
- troisièmement, le caractère thrombogène de la membrane semi-perméable composite est très nettement réduit si on le compare à celui de la membrane serm-perméable support qu'elle renferme.

Selon une variante de l'invention, le polymère cationique est une polyamine, de préférence hydrophile, et portant des groupements amines primaires, secondaires, tertiaires ou quaternaires. De préférence, le polymère cationique est une polyéthylèneimine (PEI). La quantité de PEI déposée et fixée peut varier entre environ 1 mg et environ 30 mg par m² de membrane (bornes incluses).

L'agent anticoagulant convenant à l'invention ne doit pas être toxique.

Selon une autre variante de l'invention, l'agent anticoagulant porteur de groupements anioniques appartient à la famille des glycoaminoglycanes ayant une activité anticoagulante. De préférence, cet agent est essentiellement constitué d'héparine (fractionnée ou non). La quantité d'héparine déposée et fixée peut varier entre environ 200 UI et environ 20.000 UI par m² de membrane (bornes incluses), de préférence entre environ 500 UI et environ 10.000 UI par m² de membrane (bornes incluses). La quantité d'héparine fixée est choisie en fonction du type de traitement auquel l'échangeur est destiné :
- traitement intermittent (séance de 3 à 6 heures) pour des patients souffrant d'insuffisance rénale chronique ou aiguë ou traitement continu (12 à 96 heures) pour des patients souffrant d'insuffisance rénale aiguë ;
- traitement au cours duquel aucun agent anticoagulant n'est injecté dans le circuit vasculaire du patient.

La quantité d'héparine fixée est inférieure à la quantité d'héparine qui est injectée au patient lors d'un traitement classique (laquelle est l'ordre de 7.500 à 11.000 UI ou supérieure dans le cas où la membrane est rincée avec une solution d'héparine avant la mise en circulation du sang dans le circuit extracorporel).

L'invention est adaptée à la préparation de membranes semi-perméables composites à partir d'une membrane semi-perméable support constituée essentiellement par un polyacrylonitrile, porteur de groupements anioniques ou anionisables choisis parmi les groupements sulfonique, phosphorique, carboxylique, sulfurique, phosphorique, et parmi les groupements salifiés correspondants.

De préférence, les groupements anioniques ou anionisables du polyacrylonitrile sont des groupements sulfoniques acides ou des groupements sulfoniques salifiées.

Avantageusement, la membrane semi-perméable support est constituée essentiellement de copolymère d'acrylonitrile et de méthallylsulfonate de sodium, tel que le copolymère de dénomination commerciale AN69, fabriqué par HOSPAL, avec lesquels les meilleures performances ont été atteintes.

La membrane semi-perméable composite peut être sous la forme d'une membrane plane ou d'un faisceau de fibres creuses.

De préférence, l'échangeur, qui comprend la membrane semi-perméable composite selon l'invention, est stérilisé et prêt à l'emploi. Ainsi, il ne requiert pas de manipulation spéciale de la part de son utilisateur.

Dans le cas où le polymère cationique est la PEI, l'étape (c) du procédé selon la revendication 1 peut être réalisée selon les conditions suivantes :
◆ concentration en PEI: 0,04 à 20 g/l.
◆ milieu : eau ; eau glycérinés ; tampons salins ; solutions salines
◆ pH : 5 à 12
◆ débits de traitement (cas du traitement de la membrane par circulation dans l'appareil) : 50 à 500 mL/min.
◆ durée : 1 à 30 minutes
◆ circuit ouvert ou circuit fermé
◆ dans ces conditions, la concentration surfacique en PEI est comprise entre 1 et 30 mg/m².

Dans le cas où l'agent anticoagulant est l'héparine, l'étape (e) du procédé selon la revendication 1 peut être réalisée selon les conditions suivantes :
◆ concentration en héparine : 1 à 100 UI/mL
◆ milieu : eau ; eau glycérinée ; tampons salins ; solution saline
◆ pH : 5 à 10
◆ débits de traitement (cas du traitement de la membrane par circulation dans l'appareil) 50 à 500 mL/min.
◆ durée : 1 à 30 minutes
◆ circuit ouvert ou circuit fermé
◆ dans ces conditions, la concentration surfacique en héparine est comprise entre 200 et 20.000 Ul/m², de préférence entre 500 et 10.000 Ul/m².

Eventuellement, la membrane plane ou le faisceau de fibres creuses est glycériné(e) à l'issue de l'étape (a), d'où la nécessité de déglycériner avant d'entreprendre l'étape (c).

Eventuellement, on rince la membrane semi-perméable pour éliminer l'excédent de polymère cationique fixé, soit après l'étape (c) quand l'étape (c) est réalisée avant l'étape (b), ou après l'étape (d).

Eventuellement, on rince la membrane semi-perméable pour éliminer l'excédent d'agent anticoagulant non fixé, soit après l'étape (e) quand l'étape (e) est réalisée avant l'étape (b), ou après l'étape (f).

Eventuellement, on reglycérine la membrane semi-perméable selon le cas, à l'issue de l'étape (e) ou (f), après les éventuelles étapes de rinçage.

Dans le cadre de l'invention, la stérilisation de l'échangeur, sans effet significatif sur la membrane semi-perméable composite, pourra être la stérilisation par irradiation, en particulier par irradiation gamma ou la stérilisation à l'oxyde d'éthylène. La stérilisation de l'échangeur peut être mise en oeuvre à deux moments déterminés du procédé de fabrication de l'échangeur.

Selon une première variante, on stérilise l'échangeur lorsque la membrane semi-perméable à base de polyacrylonitrile, porteur de groupements anioniques ou anionisables, est revêtue dudit polymère cationique, puis on effectue, de façon extemporanée, le traitement à l'aide de la solution contenant au moins un agent anticoagulant.

Selon une seconde variante, on stérilise l'échangeur lorsque la membrane semi-perméable à base de polyacrylonitrile, porteur de groupements anioniques ou anionisables, est revêtue dudit polymère cationique et dudit agent anticoagulant.

D'autres caractéristiques et avantages de l'invention apparaîtront à la lecture des exemples qui suivent. On se reportera également aux dessins et figures annexes sur lesquels :
- la figure 1 représente les modalités de préparation d'un polymère cationique, tel que la polyéthylèneimine (PEI), par ultrafiltration ;
- la figure 2 représente l'évolution de la concentration de PEI dans l'ultrafiltrat ;
- la figure 3 représente la distribution des masses moléculaires en poids de la PEI non fractionnée, notée PEI P, (LUPASOL P, de BASF) et de la PEI fractionnée ;
- la figure 4 représente l'évolution du temps de coagulation (TCK) du sang de moutons soumis à un test de circulation extracorporelle à l'aide d'un dialyseur à membrane plane AN69 traité avec de la PEI fractionnée, stérilisé gamma, puis traité extemporanément avec de l'héparine ;
- la figure 5 représente l'évolution du temps de coagulation (TCK) du sang de moutons soumis à un test de circulation extracorporelle à l'aide d'un dialyseur à fibres creuses AN69 traité avec de la PEI fractionnée, puis hépariné, et enfin stérilisé gamma avant utilisation.

Les méthodes de dosage utilisées pour évaluer les membranes semi-perméables décrites sont les suivantes :

### Traitement des échantillons de sang préalablement aux dosages

Le sang prélevé, en sortie des dialyseurs, au cours des séances de dialyse des exemples qui suivent est mis immédiatement en présence de citrate de sodium qui, par chélation des ions calcium, stoppe toute activité de coagulation. L'échantillon est ensuite centrifugé à température ambiante à 3000 tours par minute, durant 15 minutes. Le plasma surnageant est recueilli et conservé à -20° C jusqu'au moment du dosage.

### Détermination du temps de coagulation (TCK pour Temps de Céphaline Kaolin)

La détermination du TCK est réalisée au moyen du kit de dosage commercialisé sous la dénomination C.K. PREST® par la société DIAGNOSTICA STAGO.

Le TCK permet d'apprécier l'allongement du temps de coagulation d'un plasma citraté occasionné par le déficit en certains facteurs de la coagulation ou par la présence d'un anticoagulant comme l'héparine. Dans ce dernier cas, l'allongement du temps de coagulation est proportionnel à la quantité d'héparine présente. La détermination du TCK permet donc d'apprécier le niveau d'anticoagulation du sang. La méthode de mesure de ce temps de coagulation (exprimé en seconde) est connue, elle est effectuée après recalcification et ajout d'un activateur.

### Dosage de l'héparine en milieu non plasmatique

L'héparine est déterminée dans les milieux simples (eau et électrolytes) par spectrophotométrie après formation d'un complexe avec l'azure A (absorption maximale à 630 nm).

### Test de circulation extracorporelle sur un mouton :

Les dialyseurs testés dans les exemples sont rincés par une solution de sérum physiologique (héparinée ou non) mise en circulation dans le compartiment sang (2 L à 200 mL/min.). Le compartiment dialysat est rempli par ultrafiltration. Selon le cas, le sang du mouton est anticoagulé par injection d'héparine dans la veine jugulaire cinq minutes avant le début de la séance. La circulation sanguine extracorporelle est réalisée au débit de 200 mL/min. à l'aide d'une pompe BSM22 HOSPAL (accès en carotide et restitution en jugulaire). Les pressions d'entrée et de sortie sont enregistrées. La circulation est stoppée lorsque la pression artérielle dépasse 500 mmHg au niveau de l'entrée du dialyseur, traduisant une coagulation dans le circuit.

### Exemple 1

Cet exemple décrit un procédé de préparation d'un polymère cationique, en l'occurrence une polyéthylèneimine (PEI), visant à éliminer, par fractionnement, les plus petites chaînes de polymères (de faible encombrement stérique) pouvant pénétrer dans les pores de la membrane semi-perméable à traiter, et la traverser.

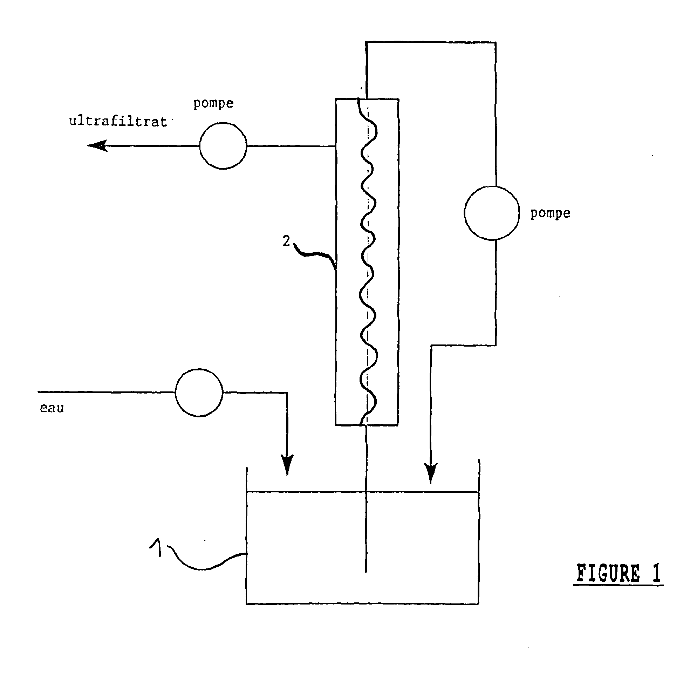

La figure 1 illustre les modalités de préparation de la PEI qui comprend les étapes suivantes :
a- préparation, dans un bac 1, d'une solution de 1,5 litre d'une solution de PEI de masse moléculaire en poids 750 k Dalton (LUPASOL P de BASF), à 50 g par litre, dans l'eau distillée ;
b- circulation, en circuit fermé, de cette solution dans le compartiment sang d'un dialyseur 2 à fibres creuses (dénomination commerciale FILTRAL 16, fabriqué par la société HOSPAL INDUSTRIE, France), équipé d'une membrane (surface utile de 1,6 m²) en AN69 (copolymère d'acrylonitrile et de méthallylsulfonate de sodium), au débit de 300 ml par minute ;
c- simultanément à l'étape b, ultrafiltration au débit de 60 ml par minute avec apport d'eau dans le bac 1, au même débit.

La durée de la préparation est de 156 minutes.

Le dosage de la PEI présente dans l'ultrafiltrat est déterminé dans l'eau par spectrophotométrie après formation d'un complexe coloré avec le thiocyanate de cobalt II (absorption maximale à 304 nm).

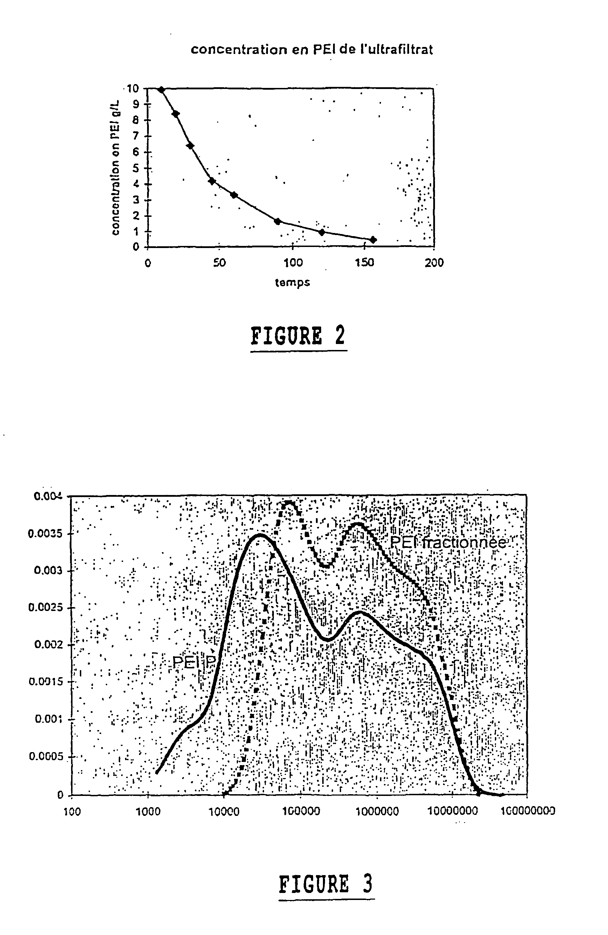

L'évolution de la concentration en PEI dans l'ultrafiltrat est donnée sur la figure 2 annexée.

Dans les conditions du procédé précité, la quantité de PEI éliminée par ultrafiltration est de 32 g, ce qui représente 43 % de la PEI de départ.

La distribution en masses moléculaires (Mw) de la PEI non fractionnée (notée PEI P) et de la PEI fractionnée est déterminée par chromatographe d'exclusion stérique (colonne ultrahydrogel de la société WATERS) et est représentée sur la figure 3 annexée. Sur la figure 3, on voit que la masse molaire des plus petites chaînes de PEI fractionnée est de l'ordre de 10.000 g/mole.

### Exemples 2

### Exemple de référence 2a

Un dialyseur à membrane plane en AN69 (dénomination commerciale CRYSTAL 4000, fabriqué par HOSPAL INDUSTRIE, France), ayant une surface utile de 1,5 m² et stérilisé par irradiation gamma, est rincé par circulation dans le compartiment sang de 2 litres de sérum physiologique contenant 5.000 UI d'héparine non fractionnée.

Un circuit extracorporel de sang comprenant le dialyseur est ensuite connecté au circuit vasculaire d'un mouton. Aucun anticoagulant n'est injecté dans le sang du mouton.

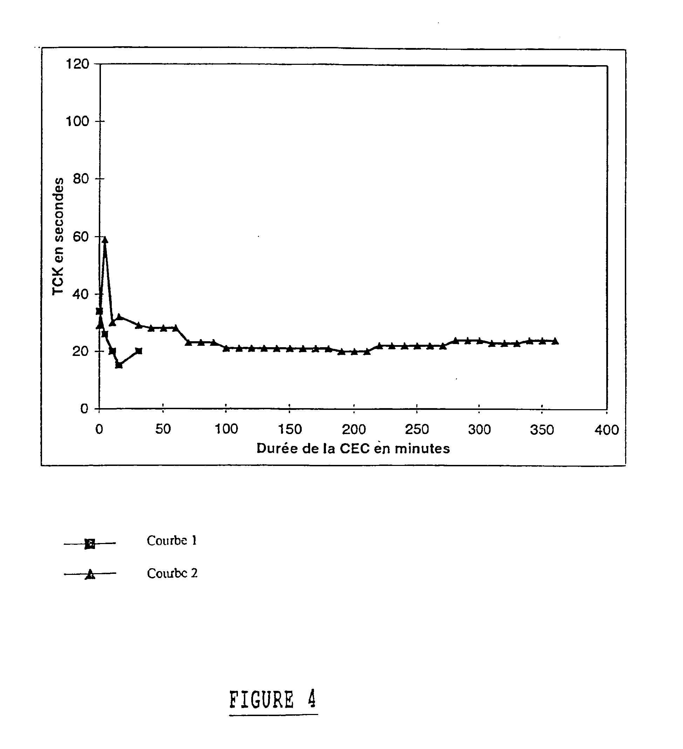

Sur la figure 4, la courbe 1 donne l'évolution du TCK pendant les 30 minutes de circulation du sang. La coagulation est intervenue au bout de 30 minutes.

### Exemple 2b selon l'invention

Un dialyseur a été fabriqué conformément à l'invention par la société HOSPAL INDUSTRIE (France). Une face d'une membrane plane en AN69, ayant une surface utile de 1,5 m², est traitée par pulvérisation de PEI fractionnée (voir exemple 1) à la concentration de 5 g/kg dans un mélange eau/glycérol 40/60 en masse. La quantité de PEI fractionnée déposée est de 10 mg/m².

Cette membrane est montée dans un dialyseur de manière que la face traitée soit orientée vers le compartiment sang du dialyseur.

Le dialyseur est ensuite stérilisé par irradiation gamma (36 K Gy).

Juste avant son utilisation, le dialyseur stérilisé est rincé à l'aide d'une solution de sérum physiologique et d'héparine non fractionnée comme indiqué dans l'exemple de référence 2a.

Un circuit extracorporel de sang comprenant le dialyseur est ensuite connecté au circuit vasculaire d'un mouton. Aucun anticoagulant n'est injecté dans le sang du mouton.

Le sang du mouton a pu être mis en circulation pendant 6 heures dans le circuit extracorporel sans qu'il se produise de coagulation (l'arrêt après 6 heures est volontaire et ne correspond pas à une coagulation du sang dans le circuit). La courbe 2 de la figure 4 donne le niveau de TCK pendant toute la durée de la circulation. Le TCK reste à une valeur normale témoignant une absence de libération d'héparine, alors que, habituellement, un TCK supérieur à 100 secondes est nécessaire pour le bon déroulement d'un traitement de sang dans un circuit de circulation extracorporelle.

### Exemples 3

### Exemple de référence 3a

Un dialyseur (dénomination commerciale FILTRAL 20, fabriqué par HOSPAL INDUSTRIE, France), équipé d'un faisceau de fibres creuses AN69 de surface utile de 2 m², stérilisé à l'oxyde d'éthylène, est rincé par circulation dans le compartiment sang de 2 litres de sérum physiologique contenant 10.000 UI d'héparine non fractionnée.

Un circuit extracorporel de sang comprenant le dialyseur est ensuite connecté au circuit vasculaire d'un mouton après que 5.000 UI d'héparine non fractionné ont été préalablement injectés au mouton.

La coagulation du sang dans le circuit extracorporel est intervenue après que le sang y a circulé 2 heures.

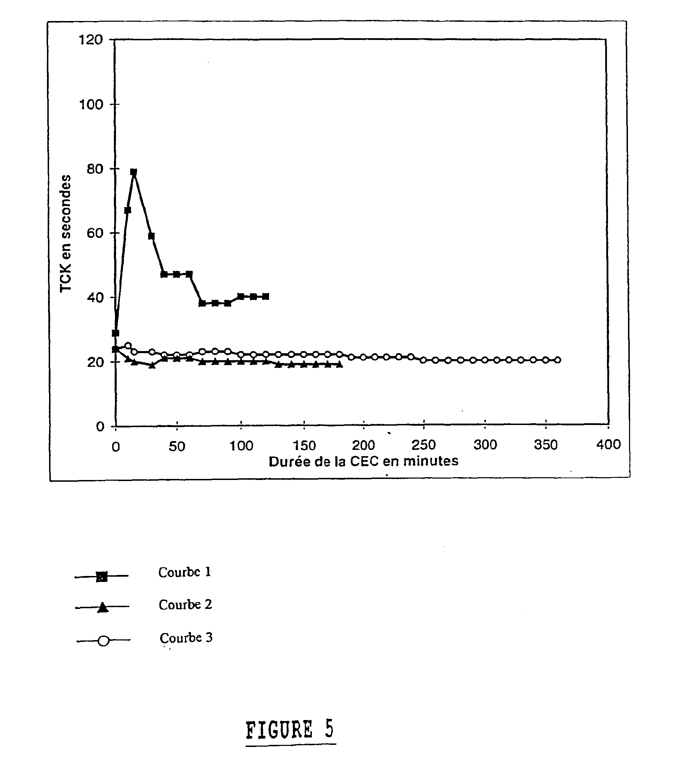

L'évolution du taux de TCK est représentée sur la figure 5, par la courbe 1.

### Exemple 3b selon l'invention

Un dialyseur (dénomination commerciale NEPHRAL 300, fabriqué par HOSPAL INDUSTRIE, France), équipé d'un faisceau de fibres creuses AN69 de surface utile de 1,3 m², est traité par circulation dans le compartiment sang d'une solution de PEI fractionnée préparée comme dans l'exemple 1 (1 g/L dans l'eau, circuit fermé sur 200 mL, 5 minutes, 200 mUmin.). Ce dialyseur subit un second traitement par circulation dans le même compartiment d'une solution d'héparine non fractionnée (5 UI/mL dans une solution phosphatée (Na2HPO₄ à 10 mM), circuit fermé sur 3 L, 200 mL/min., 5 ou 30 min.). Le dialyseur est ensuite purgé à l'air (0,3 bar pendant 30 s). Dans ces conditions, la quantité de PEI fixé est de l'ordre de 15 mg/m², la quantité d'héparine fixé est de 2.500 et 6.800 UI/m² (mesurée selon la méthode de dosage de l'héparine en milieu non plasmatique).

Ce dialyseur est stérilisé par irradiation gamma.

Juste avant l'utilisation du dialyseur, 2 litres de sérum physiologique sont mis en circulation dans le compartiment sang pour le rincer. Un circuit extracorporel de sang comprenant le dialyseur est ensuite connecté au circuit vasculaire d'un mouton. Aucun anticoagulant n'est injecté dans le sang du mouton.

La circulation du sang dans le circuit extracorporel a pu être maintenue sans injection d'héparine dans le circuit extracorporel pendant 3 et 6 heures sans qu'il se produise de coagulation et les courbes 2 et 3 de la figure 5 indiquent que les niveaux de TCK sont restés normaux.

### Exemples 4

Les exemples 4 correspondent à des séries d'essais cliniques réalisés avec des dialyseurs conformes ou non à la présente invention.

### Exemples comparatifs 4a et 4b

4a) Des dialyseurs, de dénomination commerciale NEPHRAL 300, non traités, sont utilisés dans une série d'essais cliniques réalisés dans les conditions suivantes :
   - 6 patients ;
   - 108 séances d'hémodialyse pendant 4 heures avec ajout d'héparine ;
   - préalablement à chaque séance d'hémodialyse, le dialyseur est rincé par circulation de 1 litre d'une solution de sérum physiologique contenant 5.000 UI d'héparine dans le compartiment sang du dialyseur ;
   - à la fin de chaque séance d'hémodialyse, le sang est restitué au patient par un rinçage du compartiment sang du dialyseur avec 1 litre de sérum physiologique.
4b) Des dialyseurs, de dénomination commerciale NEPHRAL 300, ont été traités successivement de la façon suivante :
   - circulation de 1 litre d'eau, avec un débit de 200 ml/min, dans le compartiment sang du dialyseur afin d'éliminer la glycérine présente dans les fibres creuses ;
   - circulation de 1 litre d'une solution de PEI de masse moléculaire moyenne en poids 750 K Dalton (LUPASOL P de BASF), non fractionnée, dans le compartiment sang du dialyseur ;
   - purge de l'air ;
   - stérilisation par irradiation gamma.
   - préalablement à chaque séance d'hémodialyse, le dialyseur est rincé par circulation de 1 litre d'une solution de sérum physiologique contenant 5.000 UI d'héparine dans le compartiment sang du dialyseur ;
   - à la fin de chaque séance d'hémodialyse, le sang est restitué au patient par un rinçage du compartiment sang du dialyseur avec 1 litre de sérum physiologique.

Une série de 27 séances d'essais cliniques est réalisée avec ces dialyseurs.

### Résultats des séries d'essais cliniques (4a) et (4b)

Après restitution du sang, les dialyseurs sont évalués visuellement, sur une échelle de 1 à 4 :
- note 1 (mauvaise) : la couleur de l'ensemble du dialyseur est rouge et témoigne d'une coagulation importante du sang dans la membrane semi-perméable ;
- note 2 (moyenne) : la couleur d'environ la moitié du dialyseur est rouge ;
- note 3 (bonne) : seulement quelques traces rouges dans le dialyseur ;
- note 4 (excellente) : le dialyseur n'est pas rouge.

| | **Exemple 4a** | **Exemple 4b (*)** |
|---|---|---|
| Valeur moyenne sur l'ensemble des séances de la quantité d'héparine ajoutée, sans compter la quantité d'héparine fixée au moment du rinçage (valeur mini, valeur maxi) (**) | 6200 | 8400 |
| | (mini: 3000) | (min : 4500) |
| | (maxi : 8000) | (maxi : 12000) |
| Valeur moyenne sur l'ensemble des séances de l'évaluation visuelle des dialyseurs (valeur mini, valeur maxi) | 3,0 | 1,9 |
| | (mini : 2) | (mini : 1) |
| | (maxi : 4) | (maxi : 3) |

| | | |
|---|---|---|
| (*) la quantité de PEI non fractionnée fixée est de l'ordre de 100 mg/m² et la quantité d'héparine fixée, au moment du rinçage du dialyseur, est de l'ordre de 2000 UI/m² de membrane. (**) la quantité d'héparine ajoutée permet de maintenir le TCK entre 90 et 120 s. | | |

### Conclusion

Le traitement avec une PEI non fractionnée conduit à une consommation (adsorption) importante d'héparine. En outre, l'héparine adsorbée n'est pas active puisque les dialyseurs ainsi traités des exemples (4b) conduisent à des problèmes de coagulation.

### Exemples 4c, selon la présente invention

Une membrane plane en AN69 est traitée par pulvérisation de PEI fractionnée, préparée dans les conditions énoncées ci-dessus dans l'exemple 1, à raison de 10 mg/m² de membrane.

Des dialyseurs de type CRYSTAL sont montés avec cette membrane (1,25 m² de surface utile de membrane par dialyseur) et sont stérilisés par irradiation gamma.

Une série d'essais cliniques est réalisée avec ces dialyseurs dans les conditions suivantes :
• 13 patients ;
• 256 séances d'hémodialyse ;
• préalablement à chaque séance d'hémodialyse, le dialyseur est rincé par circulation de 2 litres d'une solution de sérum physiologique contenant 5.000 UI d'héparine par litre, dans le compartiment sang du dialyseur ;
• séances d'hémodialyse pendant 4 heures avec ou sans ajout d'héparine (voir tableau) ;
• à la fin de chaque séance d'hémodialyse, le sang est restitué au patient par un rinçage du compartiment sang du dialyseur avec 1 litre de sérum physiologique.

| | | | | | | | |
|---|---|---|---|---|---|---|---|
| Valeur moyenne sur l'ensemble des exemples (4c) de la quantité d'héparine ajoutée, sans compter la quantité d'héparine fixée au moment du rinçage (*) | 6000 | 5000 | 4000 | 3000 | 2000 | 1000 | 0 |
| Valeur moyenne sur l'ensemble des séances de l'évaluation visuelle des dialyseurs (nombre de séances) | 4 (39) | 4 (39) | 4 (38) | 4 (35) | 4 (31) | 4 (28) | 4 (28) |
| | | | 2 (1) | 2 (2) | 2 (4) | 2 (4) | 2 (4) |
| | | | | 1 (1) | | 1 (1) | 1 (1) |

| | | | | | | | |
|---|---|---|---|---|---|---|---|
| (*) La quantité d'héparine fixée au moment du rinçage est de l'ordre de 1.500 UI/m² de membrane. | | | | | | | |

### Conclusion

Le traitement avec une PEI fractionnée, suivi d'un traitement avec l'héparine, permet d'effectuer des dialyses avec des quantités réduites d'anticoagulant injectées chez le patient.

### Remarques

Dans les exemples comparatifs (4a) et (4b), le TCK est maintenu entre 90 et 120 s.

Dans les exemples (4c), c'est la dose d'héparine injectée qui est fixée (entre 0 et 6000) et, dans tous les cas, le TCK en fin de séance est inférieur à 50, ce qui est un avantage considérable pour le patient (plus d'héparine circulante en fin de dialyse donc plus de risque hémorragique).

Après restitution du sang, les dialyseurs de cette série d'essais cliniques sont évalués précédemment dans les conditions énoncées ci-dessus pour les exemples (4a) et (4b).

## Revendications

1. Procédé pour réduire le caractère thrombogène d'un échangeur pour le traitement du sang ou du plasma par circulation extracorporelle comprenant deux compartiments séparés par une membrane semi-perméable ayant une surface orientée vers un premier compartiment destiné à la circulation de sang ou de plasma, le procédé comprenant les étapes successives suivantes:
(a) préparer une membrane semi-perméable sous la forme d'une membrane plane ou d'un faisceau de fibres creuses, à partir d'une solution de polyacrylonitrile porteur de groupements anioniques ou anionisables;
(b) assembler les divers composants de l'échangeur, en particulier monter la membrane semi-perméable ou un faisceau de fibres creuses dans un boîtier;
(c) préparer une solution contenant au moins un polymère cationique, porteur de groupements cationiques aptes à former une liaison ionique avec les groupements anioniques ou anionisables du polyacrylonitrile, le polymère cationique ne comprenant que des chaînes de polymère de taille suffisante pour ne pas traverser la membrane semi-perméable, et porter cette solution au contact de la surface de la membrane semi-perméable destinée à être mise en contact avec le sang ou le plasma, l'étape (c) pouvant être réalisée avant ou après l'étape (b) ;
(d) dans le cas où l'étape (c) est réalisée postérieurement à l'étape (b), purger l'échangeur de la solution contenant le polymère cationique;
(e) préparer une solution contenant au moins un agent anticoagulant à l'état dissous, porteur de groupements anioniques aptes à former une liaison ionique avec les groupements cationiques dudit polymère cationique et porter cette solution au contact de la surface de la membrane semi-perméable destinée à être mise en contact avec le sang, l'étape (e) étant mise en oeuvre après l'étape (c), mais avant ou après l'étape (b) ;
(f) dans le cas où l'étape (e) est réalisée postérieurement à l'étape (b), purger l'échangeur de la solution contenant l'agent anticoagulant ;
(g) stériliser l'échangeur lorsque la membrane semi-perméable à base de polyacrylonitrile, porteur de groupements anioniques ou anionisables, est revêtue dudit polymère cationique et dudit agent anticoagulant.

2. Procédé selon la revendication 1, **caractérisé en ce que** on rince la membrane semi-perméable pour éliminer l'excédent de polymère cationique non fixé, soit après l'étape (c) quand l'étape (c) est réalisée avant l'étape (b), ou après l'étape (d).

3. Procédé selon la revendication 1 ou 2, **caractérisé en ce que** on rince la membrane semi-perméable pour éliminer l'excédent d'agent anticoagulant non fixé, soit après l'étape (e) quand l'étape (e) est réalisée avant l'étape (b), ou après l'étape (f).

4. Procédé selon la revendication 1, **caractérisé en ce que** le polymère cationique est préparé par ultrafiltration au moyen d'une membrane semi-perméable qui est identique à la membrane semi-perméable support ou qui possède un seuil de coupure égal ou supérieur à celui de la membrane semi-perméable support, afin d'éliminer les chaînes de polymère cationique pouvant traverser la membrane semi-perméable support.

5. Procédé selon une des revendications précédentes où le polymère cationique est une polyéthylèneimine.

6. Procédé selon la revendication précédente où la polyéthylèneimine déposée et fixée varie entre environ 1 mg et environ 30 mg par m² de membrane, bornes incluses.

7. Procédé selon une des revendications précédentes où l'agent anticoagulant porteur de groupements anioniques appartient à la famille des glycoaminoglycanes ayant une activité anticoagulante.

8. Procédé selon la revendication précédente où l'agent anticoagulant est essentiellement constitué d'héparine.

9. Procédé selon la revendication précédente où l'héparine déposée et fixée varie entre environ 200UI et environ 20000 UI par m² de membrane, de préférence entre 500UI et 10000UI par m² de membrane.

## Claims

1. A method for reducing the thrombogenic character of an exchanger for blood or plasma treatment by extracorporeal circulation, comprising two compartments separated by a semi-permeable membrane having a surface oriented towards a first compartment intended for blood or plasma circulation, the method comprising the following successive steps:
(a) preparing a semi-permeable membrane in the form of a flat membrane or of a bundle of hollow fibers, starting from a solution of polyacrylonitrile carrying anionic or anionizable groups;
(b) assembling the various components of the exchanger, in particular fitting the semi-permeable membrane or a bundle of hollow fibers in a case;
(c) preparing a solution containing at least one cationic polymer carrying cationic groups apt to form a ionic bond with the anionic or anionizable groups of polyacrylonitrile, the cationic polymer comprising only polymer chains whose size is sufficient for them not to get through the semi-permeable membrane, and bringing this solution in contact with the surface of the semi-permeable membrane intended to be brought in contact with blood or plasma, wherein step (c) can be carried out before or after step (b);
(d) if step (c) is carried out after step (b), purging the exchanger of the solution containing the cationic polymer;
(e) preparing a solution containing at least one anticoagulant in a dissolved state, carrying anionic groups apt to form a ionic bond with the cationic groups of said cationic polymer, and bringing this solution in contact with the surface of the semi-permeable membrane intended to be brought in contact with blood, step (e) being implemented after step (c) but before or after step (b);
(f) if step (e) is carried out after step (b), purging the exchanger of the solution containing the anticoagulant;
(g) sterilizing the exchanger when the semi-permeable membrane based on polyacrylonitrile carrying anionic or anionizable groups is coated with said cationic polymer and said anticoagulant.

2. The method according to claim 1, **characterized in that** the semi-permeable membrane is rinsed so as to eliminate the excess of unbound cationic polymer, either after step (c) when step (c) is carried out before step (b), or after step (d).

3. The method according to claim 1 or 2, **characterized in that** the semi-permeable membrane is rinsed so as to eliminate the excess of unbound anticoagulant, either after step (e) when step (e) is carried out before step (b), or after step (f).

4. The method according to claim 1, **characterized in that** the cationic polymer is prepared by ultrafiltration using a semi-permeable membrane identical to the semi-permeable support membrane or having the same or a higher cut-off threshold with respect to the semi-permeable support membrane, in order to eliminate cationic polymer chains that might get through the semi-permeable support membrane.

5. The method according to one of the preceding claims, wherein the cationic polymer is a polyethyleneimine.

6. The method according to the preceding claim, wherein the polyethyleneimine deposited and bound varies from about 1 mg to about 30 mg pro sqm of membrane, including the end portions.

7. The method according to one of the preceding claims, wherein the anticoagulant carrying anionic groups belongs to the family of glycoaminoglycans having an anticoagulant activity.

8. The method according to the preceding claim, wherein the anticoagulant basically consists of heparin.

9. The method according to the preceding claim, wherein the heparin deposited and bound varies from about 200 IU to about 20,000 IU pro sqm of membranes, preferably from 500 IU to 10,000 IU pro sqm of membrane.

## Patentansprüche

1. Verfahren zur Verminderung des thrombogenen Charakters eines Austauschers für Blut- oder Plasmabehandlung durch extrakorporale Zirkulation, umfassend zwei Abteile, die durch eine semipermeable Membrane voneinander getrennt sind, die eine Fläche aufweist, die zu einem ersten Abteil zur Blut- oder Plasmazirkulation ausgerichtet ist, wobei das Verfahren die folgenden Schritte in Reihenfolge umfasst:
(a) Vorbereiten einer semipermeable Membran in Form einer flachen Membran oder eines Hohlfaserbündel von einer Lösung von Polyacrylnitril mit anionischen oder anionisierbaren Gruppierungen;
(b) Montage der verschiedenen Bestandteile des Austauschers, insbesondere Einbau der semipermeable Membran oder eines Hohlfaserbündels in ein Gehäuse;
(c) Vorbereiten einer Lösung enthaltend mindestens ein kationisches Polymer mit kationischen Gruppierungen, die eine ionische Bindung mit den anionischen oder anionisierbaren Gruppierungen des Polyacryinitrils bilden können, wobei das kationische Polymer nur Polymerketten enthält, deren Größe es ermöglicht, durch die semipermeable Membran nicht zu gehen, und Kontaktieren dieser Lösung mit der Fläche der semipermeable Membran, die zum Kontakt mit Blut oder Plasma vorgesehen ist, wobei der Schritt (c) vor oder nach dem Schritt (b) durchgeführt werden kann;
(d) wenn der Schritt (c) nach dem Schritt (b) durchgeführt wird, Spülen des Austauschers von der das kationische Polymer enthaltenden Lösung;
(e) Vorbereiten einer Lösung enthaltend mindestens einen Antikoagulationsstoff in gelöstem Zustand mit anionischen Gruppierungen, die eine ionische Bindung mit den kationischen Gruppierungen des benannten kationischen Polymers bilden können, und Kontaktieren dieser Lösung mit der Fläche der semipermeable Membran, die zum Kontakt mit Blut vorgesehen ist, wobei der Schritt (e) nach dem Schritt (c) aber vor oder nach dem Schritt (b) durchgeführt werden kann;
(f) wenn der Schritt (e) nach dem Schritt (b) durchgeführt wird, Spülen des Austauschers von der den Antikoagulationsstoff enthaltenden Lösung;
(g) Sterilisieren des Austauschers, wenn die semipermeable Membran auf Basis von Polyacrylnitril mit anionischen oder anionisierbaren Gruppierungen mit dem benannten kationischen Polymer und dem benannten Antikoagulationsstoff beschichtet ist.

2. Verfahren nach dem Anspruch 1, **dadurch gekennzeichnet, daß** die semipermeable Membran entweder nach dem Schritt (c), wenn der Schritt (c) vor dem Schritt (b) durchgeführt wird, oder nach dem Schritt (d) abgespült wird, um den Überschuß von ungebundenem kationischen Polymer zu beseitigen.

3. Verfahren nach dem Anspruch 1 oder 2, **dadurch gekennzeichnet, daß** die semipermeable Membran entweder nach dem Schritt (e), wenn der Schritt (e) vor dem Schritt (b) durchgeführt wird, oder nach dem Schritt (f) abgespült wird, um den Überschuß von ungebundenem Antikoagulationsstoff zu beseitigen.

4. Verfahren nach dem Anspruch 1, **dadurch gekennzeichnet, daß** das kationische Polymer durch Ultrafiltration unter Verwendung einer semipermeable Membran vorbereitet wird, die mit der semipermeablen Trägermembran identisch ist oder die dieselbe oder eine höhere Cutoff-Schwelle im Vergleich zur semipermeablen Trägermembran aufweist, um die kationischen Polymerketten, die durch die semipermeable Trägermembran gehen können, zu beseitigen.

5. Verfahren nach einem der vorhergehenden Ansprüche, worin das kationische Polymer ein Polyethylenimin ist.

6. Verfahren nach dem vorhergehenden Anspruch, worin das abgelagerte und gebundene Polyethylenimin im Bereich von ca. 1 mg bis ca. 30 mg pro m² Membran, einschließlich Grenzen, liegt.

7. Verfahren nach einem der vorhergehenden Ansprüche, worin der Antikoagulationsstoff mit anionischen Gruppierungen zur Familie der Glykoaminoglykane mit einer Antikoagulationsaktivität gehört.

8. Verfahren nach dem vorhergehenden Anspruch, worin der Antikoagulationsstoff wesentlich aus Heparin besteht.

9. Verfahren nach einem der vorhergehenden Ansprüche, worin das abgelagerte und gebundene Heparin im Bereich von ca. 200 IE bis ca. 20.000 IE pro m² Membran, bevorzugt von 500 IE bis 10.000 IE pro m² Membran, liegt.
